# EUROPÄISCHE PATENTANMELDUNG

(11) **EP 1 974 760 A1**
(43) Veröffentlichungstag der Anmeldung: **01.10.2008**
(21) Anmeldenummer: 07118207.5
(22) Anmeldetag: 10.10.2007
(51) Int. Cl.: A61M 5/315

(54) **Injektionsgerät, insbesondere Insulin-Pen**

(30) Priorität: 29.03.2007 EP 07105279
(71) Anmelder: Schmidt-Evers, Jürgen, 82211 Herrsching (DE); Ruhland, Bernd, 81377 München (DE); Micheler, Clemens, 82319 Starnberg (DE)
(72) Erfinder: Schmidt-Evers, Jürgen, 82211 Herrsching (DE); Ruhland, Bernd, 81377 München (DE); Micheler, Clemens, 82319 Starnberg (DE)
(74) Vertreter: Schmidt-Evers, Jürgen

(57) **Zusammenfassung**

Die Erfindung betrifft einen Pen-Injektor für ein flüssiges Injektionsmittel (7), insbesondere Insulin. Das Injektionsmittel (7) befindet sich in einem zylindrischen Behälter mit einem verschiebbaren Kolben (4). Der Kolben (4) weist ein Gewindeloch (4c) auf, durch das eine Gewindestange (5) hindurchgeführt ist und sich in den mit dem Injektionsmittel (7) gefüllten Teil des Behälters (3) erstreckt. Die Gewindestange (5) ist gegen von dem Behälter (3) weggerichtete Axialverschiebungen fixiert. Der Pen-Injektor weist ferner eine Freilaufkupplung (2) auf, die beim Drehen eines Drehelementes (6) die Gewindestange 5 W einer Drehrichtung mitmimmt und in der anderen Drehrichtung entkoppelt.

## Beschreibung

Die Erfindung betrifft einen Pen-Injektor für ein flüssiges Injektionsmittel, insbesondere Insulin, mit einem Gehäuse, mindestens einem Behälter zur Aufnahme des Injektionsmittels, einer Auslassöffnung an dem Behälter, einem verschiebbaren Kolben in dem Behälter, einer die Kolbenstange bildenden Gewindestange, einem von Hand zu betätigendem Drehelement, und einer Freilaufkupplung zwischen dem Drehelement und der Gewindestange.

Ein derartiger Pen-Injektor ist nach der EP 1 289 579 B1 bekannt. Die die Gewindestange bildende Kolbenstange setzt hier an Oberseite des Kolbens an, um diesen in den Behälter hineinzuschieben.

Der Erfindung liegt die Aufgabe zugrunde, einen von Hand zu betätigenden Pen-Injektor, insbesondere einer solchen zur Injektion von Insulin, zu schaffen, der sich dadurch auszeichnet, dass er besonders kurz, einfach aufgebaut und auch einfach zu bedienen ist.

Die Aufgabe ist erfindungsgemäß gelöst durch folgende Merkmale:
(a) einem Gewindeloch in dem Kolben,
(b) die Gewindestange ist durch das Gewindeloch in dem Kolben hindurchgeführt und erstreckt sich in den mit dem Injektionsmittel geführten Teil des Behälters,
(c) die Gewindestange ist gegen von dem Behälter weggerichtete Axialverschiebungen fixiert.

Durch die erfindungsgemäße Lösung wird in erster Linie der Vorteil erreicht, dass der Pen-Injektor verkürzt werden kann. Ein weiterer Vorteil ist, dass er aus nur wenigen Bauteilen herstellbar ist. Durch Drehen des Drehelementes in der einen Drehrichtung von Hand ist eine einfache und genaue Dosierung möglich. Die Injektion kann dann ebenfalls in einfacher Weise durch Drehen des gleichen Drehelementes in der anderen Richtung realisiert werden.

Nach der US 3,082,914 ist zwar ein Dispenser bekannt, bei dem die Gewindestange ebenfalls durch ein Gewindeloch des Kolbens hindurchgeführt ist und sich in den mit dem Injektionsmittel gefüllten Teil des Behälters erstreckt; hier ist die Gewindestange jedoch nicht - zumindest in der ersten Betriebsphase - gegen von dem Behälter weggerichtete Axialverschiebungen fixiert. Vielmehr soll sich die Gewindestange zunächst axial zurückbewegen, um ein Durchmischen eines Pulvers und eines Lösungsmittels zu ermöglichen. Außerdem weist der bekannte Pen-Injektor keine Freilaufkupplung auf. Das bedeutet, dass eine Dosierung durch Rückwärtsdrehen des Drehelementes nicht vorgesehen ist.

Nach der US 2,349,726 ist ein weiterer Dispenser bekannt, der zur Abgabe von pastöser Masse, beispielsweise Zahnpaste bestimmt ist. Auch hier erstreckt sich eine Gewindestange durch ein Gewindeloch in dem Kolben bis in den mit der pastösen Masse gefüllten Teil des Behälters. Bei diesem Dispenser dient jedoch das Gehäuse zur Aufnahme der pastösen Masse d.h. es ist kein extra Behälter für die pastöse Masse vorgesehen. Auch weist dieser bekannte Dispenser keine Freilaufkupplung auf. Seine Gewindestange ist auch nicht gegen von dem Behälter weggerichtete Axialverschiebungen fixiert. Sie soll vielmehr durch axiale Verschiebbarkeit gewährleisten, dass die Auslassöffnung wahlweise geöffnet oder verschlossen wird.

Nach der WO 94/15660 ist ein motorgetriebenes Infusionsgerät bekannt, bei dem sich eine Gewindestange ebenfalls durch ein Gewindeloch in dem Kolben des Behälters für das Infusionsmittel erstreckt. Hier ist die Gewindestange zwar gegen von dem Behälter weggerichtete Axialverschiebungen fixiert; es ist aber keine Freilaufkupplung vorgesehen, die eine Dosierung des nächsten Hubes von Hand erlaubt. Vielmehr ist lediglich vorgesehen, durch Drehen der Gewindestange in nur einer Richtung nacheinander Injektionen auszuführen, wobei die Dosierung elektronisch durch Vorfestlegung der Umdrehungen der Gewindestange in nur einer Richtung erfolgt.

Eine Weiterbildung des erfindungsgemäßen Pen-Injektors kann darin bestehen, dass zwischen dem Drehelement und der Gewindestange zusätzlich ein Übersetzungsgetriebe eingeschaltet ist. Damit ist es möglich, dass die Zahl der Drehungen, die für die Dosierung und die Ausführung Injektion erforderlich sind, auf einen akzeptablen Betrag reduziert werden kann. Am zweckmäßigsten ist es, wenn der Benutzer des Pen-Injektors die Injektion mit nur maximal einer Umdrehung des Drehelementes realisieren kann.

Unter der ein Merkmal der erfindungsgemäßen Merkmalskombination bildenden "Freilaufkupplung" soll ein Kupplungselement zwischen zwei drehbaren Teilen verstanden werden, das eine Drehverbindung zwischen diesen Elementen nur in einer Drehrichtung gewährleistet ist, während die Elemente in der anderen Drehrichtung entkoppelt sind.

Die Freilaufkupplung kann gemäß einer Weiterbildung der Erfindung eine Vielzahl von Raststellungen aufweisen. Dadurch ist es möglich, dass der Anwender des Pen-Injektors bei der Dosierung die Zahl der einzustellenden Einheiten akustisch aufnehmen und mitzählen kann.

Eine andere Weiterbildung kann darin bestehen, dass die Freilaufkupplung nach Art eines Speichenrades mit Radring, Radnabe und dazwischen angeordneten Speichen aufgebaut ist, wobei die Speichen geneigt zur Radialrichtung verlaufen. Die Radnabe kann von dem über dem Kolben vorstehenden Endbereich der Gewindestange gebildet oder mit diesem verbunden sein, wobei die Speichen nur mit der Radnabe oder nur mit dem Radring verbunden sind, und wobei die Speichen ein freies Ende haben, das an dem jeweils anderem Teil - Radnabe oder Radring - unter Bildung eines spitzen Winkels anliegt.

Zur Verbesserung der Kupplungseigenschaften wird ferner vorgeschlagen, dass an dem jeweils anderen Teil - Radnabe oder Radring -, an dem die freien Enden der Speichen anliegen, Rastausnehmungen und/oder - vorsprünge vorgesehen sind.

Eine andere Weiterbildung kann darin bestehen, dass das Übersetzungsgetriebe ein Planetengetriebe ist, wobei der Planetenring das Drehelement bildet, und dass das Zentralrad mit der Gewindestange über die Freilaufkupplung verbunden ist.

Das Zentralrad des Planetengetriebes kann mit dem Radring der Freilaufkupplung verbunden oder einstückig mit diesem ausgebildet sein.

Eine andere Weiterbildung kann darin bestehen, dass Drehbegrenzungsmittel für das Drehelement vorgesehen sind, die eine Drehung des Drehelementes um maximal 360 Grad erlauben.

Eine andere Weiterbildung kann darin bestehen, dass der Pen-Injektor mit Dosierungsanzeige-Mittel versehen ist. Zu dieser können auf dem Radring der Freilaufkupplung angebrachte Dosierungszahlen gehören, die durch ein Fenster in dem Gehäuse von außen sichtbar sind. Ferner können dazu Dosierungszahlen und/oder farbkodierte Sektoren gehören, die auf dem das Drehelemente bildenden Planetenring angebracht sind. Die auf dem Radring der Freilaufkupplung angebrachten Dosierungszahlen können dabei die Einer-Stellen und die auf dem Planetenring angebrachten Dosierungszahlen können die Zehner-Stellen repräsentieren.

Weiterhin wird vorgeschlagen, dass das Übersetzungsverhältnis des Übersetzungsgetriebes und die Steigung der Gewindestange so gewählt werden, dass mit einer Umdrehung des Drehelementes der gewünschte Maximalhub erreicht wird.

Eine andere Weiterbildung kann darin bestehen, dass der Kolben aus einem das Gewindeloch enthaltenden festen Kern und einer aus nachgiebigem Material bestehenden Umhüllung besteht. Auf diese Weise soll eine gute Abdichtung zwischen Gewindestange und Kolben gewährleistet werden.

Der Behälter für das Injektionsmittel kann eine Fertigkarpule sein, die die durch den Kolben geführte Gewindestange enthält, wobei der von dem Behälter weggerichtete Endabschnitt der Gewindestange über dem Kolben vorsteht.

Eine das Gehäuse betreffende Weiterbildung kann darin bestehen, dass dieses aus einem becherartigem Unterteil und einem ebenfalls becherartigen Oberteil besteht, wobei das Oberteil und das Unterteil mit ihren Öffnungen gegeneinander gerichtet und miteinander verbindbar sind. Das Unterteil dient dabei zur Aufnahme des Behälters für das Injektionsmittel, während das Oberteil zur Aufnahme der Freilaufkupplung und des Übersetzungsgetriebes dient.

Ferner kann das Gehäuse - zur Bildung eines Doppelpens - ein Unterteil aufweisen, das mindestens zwei Aufnahmeräume für je einen Behälter aufweist. In diesem Fall kann für jeden Behälter ein separates Oberteil vorgesehen werden, welches zur Aufnahme jeweils einer Freilaufkupplung und eines Übersetzungsgetriebes dient. Das Unterteil wird dann mit einem Anastomoseteil verbunden, welches die Ausgänge der Behälter zusammenführt und in einen gemeinsamen Injektionskanal münden lässt.

Ein Doppelpen ist dem Prinzip nach bereits in der WO 01/02039 A1 beschrieben. Der hier betrachtete Doppelpen zeichnet sich jedoch dadurch aus, dass sich die Elemente des vorstehend beschriebenen Einzelgerätes besonders gut auch zu seiner Realisierung eignen.

Der erfindungsgemäße Pen-Injektor kann ferner mit einem Energiespeicher- und Antriebsmechanismus versehen werden, der mit dem Drehen des Drehelementes in der die Dosierung bewirkenden Drehrichtung geladen wird und der das Drehelement zur Ausführung der Injektion in der anderen Drehrichtung dreht. Bei einem derartig ausgestaltetem Pen-Injektors kann gegebenenfalls auf das Übersetzungsgetriebe verzichtet werden.

Der Energiespeicher- und Antriebsmechanismus kann eine aufgewickelte Wendelfeder oder dgl. enthalten, die bei der die Dosierung bewirkenden Drehung des Drehringes gestreckt wird und sich bei der Ausführung der Injektion auf die ursprüngliche Länge verkürzt.

Die Wendelfeder kann auf einen Wickelkörper aufgewickelt sein, der am Gehäuse befestigt ist, wobei die Wendelfeder mit einem Ende am Drehelement und mit dem anderem Ende am Gehäuse befestigt ist.

Ferner kann der Energiespeicher- und/oder Antriebsmechanismus mit einem von Hand zu betätigendem Auslöseelement versehen sein.

Die Erfindung betrifft ferne eine Fertigkarpule für einen Pen-Injektor, der wie vorstehend beschrieben ausgebildet ist. Diese Fertigkarpule ist dadurch gekennzeichnet, dass sie eine durch den Kolben geführte Gewindestange enthält und dass der von der Karpule weggerichtete Endabschnitt der Gewindestange über dem Kolben vorsteht.

Der über dem Kolben vorstehende Endabschnitt der Gewindestange kann mit sich in axialer Richtung erstreckenden Rastausnehmung und/oder - Vorsprüngen versehen sein.

Schließlich betrifft die Erfindung noch ein Verfahren zum Betreiben eines Pen-Injektors, der wie vorstehend ausgebildet ist. Das Verfahren ist gekennzeichnet durch folgenden Schritte:
(aa) das Drehelement wird - ausgehend vom Drehanschlag - von Hand zur Dosierung des Hubes für die nächste Injektion in die Richtung gedreht, in der die Freilaufkupplung keine Drehmitnahme bewirkt,
(bb) das Drehelement wird ebenfalls von Hand oder durch einen Energiespeicher- und Antriebsmechanismus zur Ausführung der Injektion bis zum Drehanschlag in die andere Richtung gedreht, in der die Freilaufkupplung eine Drehmitnahme bewirkt.

Ausführungsbeispiele der Erfindung werden nachfolgend anhand der Zeichnungen beschrieben. Es zeigen:
- Fig. 1a: einen axialen Schnitt durch einen Pen-Injektor in Bereitschaftsstellung zur Ausführung einer Injektion,
- Fig. 1b: eine Drehbewegung am Drehelement in der einen Richtung zu Ausführung der Injektion,
- Fig. 1c: eine Drehbewegung in der anderen Richtung zur Dosierung des Hubes für die nächste Injektion,
- Fig. 1d: eine erneute Drehbewegung in der einen Richtung zur Ausführung der nächsten Injektion,
- Fig. 2: einen Schnitt A-A durch den Pen-Injektor nach Figur 1, der insbesondere das Planetengetriebe zeigt,
- Fig. 3: einen Schnitt B-B durch den Pen-Injektor nach Figur 1, der insbesondere die Freilaufkupplung zeigt,
- Fig. 4: eine perspektivische Ansicht des Radringes der Freilaufkupplung mit dem einstückig daran angeformten Zentralrad des Planetengetriebes,
- Fig. 5: eine Frontansicht auf den oberen Teil des Pen-Injektors nach Figur 1,
- Fig. 6: einen axialen Schnitt durch einen Doppel-Pen-Injektor mit zwei Injektionsmittel-Behältern,
- Fig. 7: eine Fertigkarpule (Injektionsmittel-Behälter),
- Fig. 8: eine Ansicht von oben auf den Doppel-Pen-Injektor nach Figur 6,
- Fig. 9: eine Frontansicht auf den oberen Teil des Pin-Injektors, der hier mit einem Energiespeicher- und Antriebsmechanismus versehen ist.

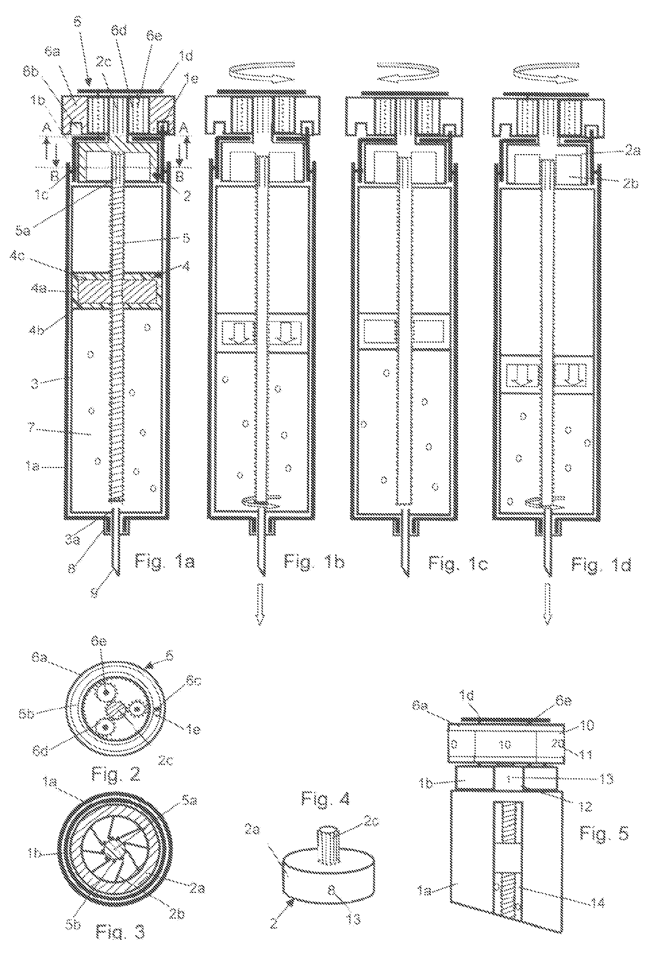

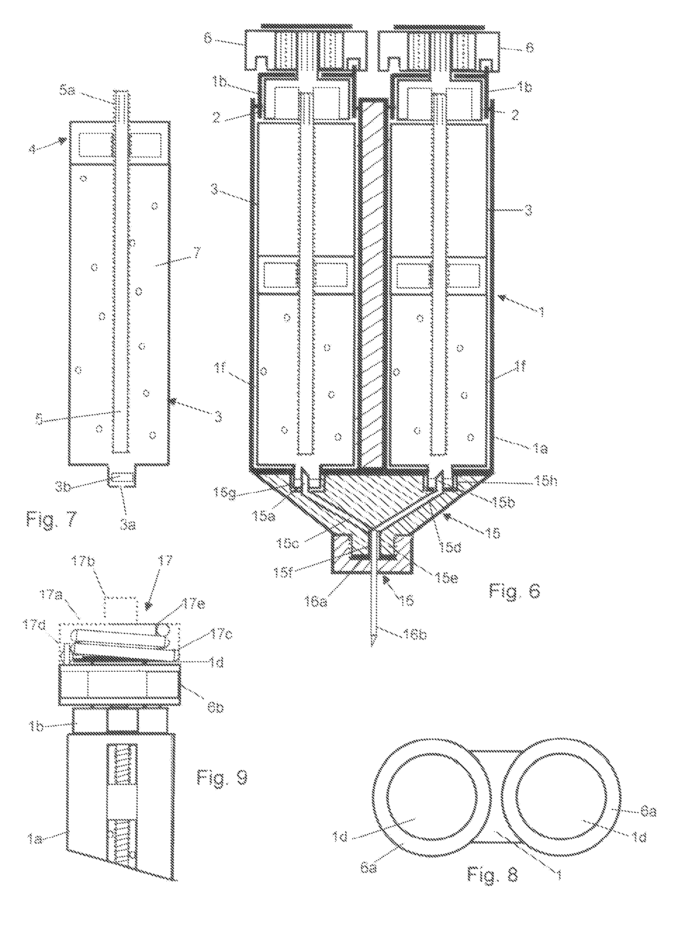

Der in Figur 1a gezeigte Pen-Injektor besteht aus einem becherartigen Gehäuse-Unterteil 1a und einem ebenfalls becherartigen Gehäuse-Oberteil 1b, die durch einen Bajonettverschluss 1c miteinander verbindbar sind. Der Gehäuse-Unterteil 1a dient zur Aufnahme eines Behälters 3, der in Figur 7 nochmals separat gezeigt ist. Der Behälter 3 ist hier eine auswechselbare Fertigkarpule. Es ist aber auch möglich, den Pen-Injektor als Einweg-Gerät auszubilden.

Der Behälter 3 enthält das Injektionsmittel 7. In dem zylindrisch ausgebildeten Behälter 3 ist ein Kolben 4 verschiebbar angeordnet. Er besteht aus einem festen Kern 4b mit einem Gewindeloch 4c und ist mit einer Ummantelung 4a aus nachgiebigem Material versehen. Letztere sorgt für eine gute Abdichtung gegenüber der Zylinderwand des Behälters 3 sowie gegenüber einer Gewindestange 5, die sich durch das Gewindeloch 4c erstreckt. Die Gewindestange 5 ist gegen von dem Behälter 3 weggerichtete Axialverschiebungen fixiert und reicht bis in den mit dem Injektionsmittel 7 gefüllten Teil des Behälters 3.

Der Oberteil 1b des Behälters trägt an seiner Oberseite ein Planetengetriebe 6 und in seinem Inneren eine Freilaufkupplung 2. Das Planetengetriebe 6 ist in Figur 2 im Schnitt gezeigt. Die Freilaufkupplung 3 ist in Figur 3 im Schnitt dargestellt.

Das Planetengetriebe 6 besteht aus einem Planetenring 6a, drei Zwischenrädern 6d und einem Zentralrad 2c. Die Zwischenräder 6d sitzen auf Drehachsen 6e, welche sich zwischen dem Oberteil 1b des Gehäuses und einer ebenfalls zum Gehäuse gehörenden Deckplatte 1d erstrecken. Der Planetenring 6a weist an seiner Unterseite eine Ringnut 6b auf, in der sich ein Drehbegrenzungssteg 6c befindet. In die Ringnut 6b ragt ein gehäusefester Anschlag 1e. Dieser bildet mit dem Drehbegrenzungssteg 6c eine Drehbegrenzung, derart, dass der Planetenring 6a nur um maximal 360° gedreht werden kann.

Die Freilaufkupplung 2 besteht aus einem kappenförmigen Teil, welches nach Art eines Rades aufgebaut ist. Dabei bildet der äußere Teil 2a den Radring, an dem sich nach innen erstreckende Speichen 2b befestigt sind. Die Speichen verlaufen nicht radial, sondern bilden mit der Radialrichtung einen spitzen Winkel zur. Sie liegen mit ihren freien Enden an Rastausnehmungen und/oder -vorsprüngen 5b an, welche sich an der Radnabe befinden. Die Radnabe ist von dem oberen Teil 5a der Gewindestange 5 gebildet. Wenn in Figur 3 der Radring 2a im Gegenuhrzeigersinn gedreht wird, so erfolgt eine Drehmitnahme der Gewindestange 5. Wenn der Radring 2a im Uhrzeigersinn gedreht wird, so sind der Radring 2a und die Gewindestange 5 entkoppelt.

Wie aus Figur 4 entnommen werden kann, ist auf dem kappenförmigen Radring 2a an dessen Oberseite einstückig das Zentralrad 2c für das Planetengetriebe 6 angeformt. Die Einstückigkeit ist zwar vorteilhaft aber nicht zwingend notwendig.

Wenn der Planetenring 6a in Figur 2 im Uhrzeigersinn gedreht wird, so wird das Zentralrad 2c mit entsprechend höherer Drehzahl ebenfalls im Uhrzeigersinn gedreht und nimmt den Radring 2a der Freilaufkupplung 2 mit, mit der Folge, dass die Speichen 2b über die Rastausnehmungen- und/oder Vorsprünge 5b rattern, was für den Benutzer des Pen-Injektors hörbar ist. Vorteilhafterweise werden gerade zehn Rastausnehmungen bzw. -vorsprünge 5b an dem oberen Endbereich 5a der Gewindestange 5 vorgesehen, so dass bei einer vollständigen Umdrehung der Radnabe 2a gerade 10 Rastgeräusche auftreten. Auf diese Weise kann auch eine Dosierung im Dezimalsystem realisiert werden.

Die Dosierung eines Injektionshubes erfolgt dadurch, dass der Planetenring 6a im Uhrzeigersinn gedreht wird, und zwar um einen Winkel, der maximal 360° ist. Geht man davon aus, dass das Übersetzungsverhältnis des Planetengetriebes 4:1 ist, so dreht sich die Gewindestange 5 bei einer Umdrehung des Planetenringes 6a viermal. Das bedeutet, dass 40 Rastgeräusche zu hören sind. Das entspricht 40 Einheiten, wenn die Gewindestange eine Steigung hat, die gerade so gewählt ist, dass bei vier Umdrehungen 40 Einheiten transportiert werden. Eine solche Dosis kann als die höchst abzugebende Dosis für Insulin festgesetzt werden. Selbstverständlich können auch höhere Maximaldosen festgesetzt werden, wobei dann die genannten Parameter entsprechend angepasst werden müssen. Wenn der Planetenring 6a um einen Winkel gedreht wird, der kleiner als 360° ist, so ist die Dosisabgabe entsprechend geringer.

In Figur 5 ist eine mögliche Dosierungsanzeige dargestellt. Dazu ist der Planetenring 6a in vier Sektoren 10 eingeteilt, die verschiedenfarbig markiert werden können. Diese vier Sektoren repräsentieren die Zehnereinheiten. Das bedeutet, dass bei einer Viertelumdrehung des Planetenringes 6a gerade zehn Einheiten abgegeben werden. Um die Einereinheiten anzuzeigen, sind auf der Außenseite der Radnabe 2a der Freilaufkupplung 2 - über den Umfang verteilt - die Zahlen 0 bis 9 eingraviert. Diese sind durch ein im Gehäuseunterteil 1b befindlichen Fenster 12 erkennbar. Dabei erscheint bei jeder Raststellung eine andere Zahl. Der jeweilige Füllstand an Injektionsmittel im Behälter ist durch ein Fenster 14 im Gehäuseunterteil 1a erkennbar.

Nachfolgend soll die Betriebsweise für den Pen-Injektor etwas genauer beschrieben werden:

Zunächst wird eine voll mit Injektionsmittel 7 gefüllte Fertigkarpule 3, wie sie in Figur 7 gezeigt ist, in den Unterteil 1a des Behälters eingesetzt. Die Fertigkarpule, die in Figur 7 etwas detaillierter gezeichnet ist als in den Figuren 1a - 1d, weist an ihrem unteren Ende einen vorspringenden Abschnitt mit dem Auslass 3a auf, in den die Injektionsnadel eingeführt wird. Dazu muss von ihr ein Septum 3b durchstochen werden. Die Fertigkarpule enthält die Gewindestange 5, welche sich durch das Gewindeloch im Kolben 5a erstreckt und mit ihrem oberen Endabschnitt 5a über dem Kolben 4 hervorsteht.

Durch Aufsetzen des Oberteiles 1b des Gehäuses auf den Unterteil 1a und durch Verriegeln des Bajonettverschlusses 1c treten die Freilaufkupplung 2 und der obere Endabschnitt 5a der Gewindestange 5 miteinander in Kontakt.

Figur 1a zeigt den Pen-Injektor nach vorheriger Dosierung in Bereitschaftsstellung für eine auszuführende Injektion.

Die Ausführung der Injektion ist in Figur 1b gezeigt. Dazu wird der als Drehelement wirkende Planetenring 6a im Gegenuhrzeigersinn soweit gedreht, bis der Drehbegrenzungssteg 6c gegen den gehäusefesten Anschlag 1e anschlägt. Durch diese Drehung wird der Radring 2a der Freilaufkupplung 2 ebenfalls im Gegenuhrzeigersinn mitgedreht, mit der Folge, dass die Speichen 2b hinter die Rastausnehmungen bzw. - Vorsprünge 5b am oberen Endabschnitt 5a der Gewindestange angreifen und die Gewindestange 5 mitnehmen.

Dass die Gewindestange 5 gegen von dem Behälter 3 weggerichtete Axialverschiebungen fixiert ist, hat zur Folge, dass der Kolben 4 nach unten transportiert wird und der Pen-Injektor Insulin ausstößt.

Figur 1c zeigt die Dosierung des Hubes für die nächst Injektion. Dazu wird der Planetenring 6a im Uhrzeigersinn gedreht, mit der Folge, dass sich der Drehbegrenzungssteg 6c von dem gehäusefesten Anschlag 1e entfernt, wobei der Drehwinkel die Dosierung bestimmt. Bei dieser Drehbewegung sind der Radring 2a der Freilaufkupplung und die Gewindestange 5 entkoppelt, d.h. es erfolgt keine Drehmitnahme.

In Figur 1d ist wiederum die Ausführung der vorbereiteten Injektion gezeigt, die wie in Figur 1b erfolgt.

Figur 6 zeigt den Aufbau eines sogenannten Doppelpens. Das Gehäuse 1 ist hier mit zwei Aufnahmeöffnungen 1f für je einen Injektions-Mittel-Behälter 3 versehen. Der Injektionsmittelbehälter kann eine Fertigkarpule sein, wie in Figur 7 gezeigt ist. Es ist aber auch möglich, dass der Doppelpen fest eingebaute Behälter enthält und somit als Einweggerät ausgebildet ist. Da sich der erfindungsgemäße Pen-Injektor durch eine geringe Zahl an Bauelementen auszeichnet, ist er besonders für die Einwegversion geeignet.

Der Doppelpen in Figur 6 unterscheidet sich von dem Einzelgerät gemäß in Figuren 1a bis 1d dadurch, dass er ein Anastomoseteil 15 aufweist. Dieses wird an den unteren Teil des Behälters 1 angesetzt. Es weist zwei Ausnehmungen 15e, 15h auf, die zur Aufnahme der mit den Auslässen 3a versehenen Endbereiche der Behälter 3 dienen. Das Anastomoseteil ist mit zwei Nadeln 15a, 15b versehen, die in die Auslassöffnungen der Behälter 3 hineinragen und das dort vorgesehene Septum durchstechen. Die Nadeln 15a, 15b sind über Zusammenführungs-Kanäle 15c, 15d mit einem Zentralkanal 15f verbunden. Letzterer befindet sich in einem Gewindeansatz 15e am Anastomoseteil 15. Auf dem Gewindeansatz 15e ist eine Nadelkappe 16 aufgeschraubt, in der sich ein Dichtring 16a befindet. Die Nadelkappe 16 weist eine Injektionsnadel 16b auf, die mit ihrem oberen Endabschnitt in den Zentralkanal 15f bis zu der Stelle hineinragt, wo die beiden Zusammenführungskanäle 15c, 15d in den Zentralkanal 15f einmünden. Auf diese Weise wird gewährleistet, dass das nach einer Injektion aus einem Behälter im Anastomoseteil verbleibende Totvolumen vernachlässigbar gering ist.

Auf das Gehäuseunterteil 1a des Doppelpen sind zwei Gehäuseoberteile 1b aufgesetzt, die in gleicher Weise ausgebildet sind, wie dies in den Figuren 1a bis 1d gezeigt und vorstehend beschrieben ist. Das bedeutet, dass alle wesentlichen Elemente, die für einen Einzel-Pin verwendet werden können, auch für einen Doppelpen Anwendung finden.

Figur 8 zeigt den Doppelpen gemäß Figur 6 von oben.

Mit dem Doppelpen können zwei verschiedene Injektionsmittel injiziert gleichzeitig oder unmittelbar aufeinander folgend durch die gleiche Kanüle injiziert werden. Der Anwender braucht sich also nur einmal zu stechen. Besonders vorteilhaft ist ein Doppelpen für Diabetiker, wenn diese zwei Insuline, beispielsweise ein schnell und ein verzögert wirkendes Insulin, in jeweils neu zu wählender Mischung mit einem Injektionsakt spritzen wollen.

In Figur 9 ist gezeigt, wie der erfindungsgemäße Pen-Injektur oder auch der Doppelpen mit einem Energiespeicher- und Antriebsmechanismus 17 versehen werden kann. Dieser besteht aus einer auf einen Wickelkörper 17e in gedehnter Form aufgewickelten Wendelfeder 17c. Diese greift mit ihrem einen Ende an der zum Gehäuse gehörenden Deckplatte 1d an und mit dem anderen Ende an dem Planetenring 6a. Durch Drehen des Planetenringes 6a in der die Dosierung bewirkenden Drehrichtung, also im Uhrzeigersinn, wird die Wendelfeder weiter gespannt. Durch Betätigen eines Auslöseelementes 17b kann dann die Injektion vollzogen werden, wobei die Wendelfeder sich auf ihre ursprüngliche Länge verkürzt und den Kolben vortreibt. Anstelle der Wendelfeder kann beispielsweise auch ein Gummiband verwendet werden.

## Patentansprüche

1. Pen-Injektur für ein flüssiges Injektionsmittel (7), insbesondere Insulin, mit
einem Gehäuse (3) zur Aufnahme des Injektionsmittels (7),
einer Auslassöffnung (3a) an dem Behälter (3),
einem verschiebbaren Kolben (4) in dem Behälter (3),
einer die Kolbenstange bildenden Gewindestange (5),
einem von Hand zu betätigenden Drehelement (6a),
und einer Freilaufkupplung (2) zwischen dem Drehelement (6a) und der Gewindestange (5),
**gekennzeichnet durch** folgende Merkmale:
(a) einem Gewindeloch (4c) in dem Kolben (4),
(b) die Gewindestange (5) ist **durch** das Gewindeloch (4c) in dem Kolben (4) hindurchgeführt und erstreckt sich in den mit dem Injektionsmittel (7) gefüllten Teil des Behälters (3),
(c) die Gewindestange (5) ist gegen von dem Behälter (3) weggerichtete Axialverschiebungen fixiert.

2. Pen-Injektor nach Anspruch 1,
**dadurch gekennzeichnet,**
**dass** zwischen dem Drehelement (6a) und der Gewindestange (5) zusätzlich ein Übersetzungsgetriebe (6) eingeschaltet ist.

3. Pen-Injektor nach Anspruch 1 oder 2,
**dadurch gekennzeichnet,**
**dass** die Freilaufkupplung (2) eine Vielzahl von Raststellungen aufweist.

4. Pen-Injektor nach einem der vorherstehenden Ansprüche,
**dadurch gekennzeichnet,**
**dass** die Freilaufkupplung (2) nach Art eines Speichenrades mit Radring (2a), Radnabe (5b) und dazwischen angeordneten Speichen (2b) aufgebaut ist,
**dass** die Speichen (2b) geneigt zur Radialrichtung verlaufen,
**dass** die Radnabe (10) von dem über dem Kolben (4) vorstehenden Endbereich (5b) der Gewindestange (5) gebildet oder mit diesem verbunden ist,
und **dass** die Speichen (2b) nur mit der Radnabe (5b) oder nur mit dem Radring (2a) verbunden sind und ein freies Ende haben, das an dem jeweils anderen Teil - Radnabe (5b) oder Radring (2a) - unter Bildung eines spitzen Winkels anliegt.

5. Pen-Injektor nach Anspruch 4, **dadurch gekennzeichnet,**
**dass** an dem jeweils anderen Teil - Radnabe (5b) oder Radring (2a) -, an dem die freien Enden der Speichen (2b) anliegen, Rastausnehmungen und/oder -vorsprünge (5c) vorgesehen sind.

6. Pen-Injektor nach einem der Ansprüche 2 bis 5,
**dadurch gekennzeichnet,**
**dass** das Übersetzungsgetriebe (6) ein Planetengetriebe ist,
**dass** der Planetenring (6a) das Drehelement bildet,
und **dass** das Zentralrad (2c) mit der Gewindestange (5) über die Freilaufkupplung (2) verbunden ist.

7. Pen-Injektor nach einem der Ansprüche 4 bis 6,
**dadurch gekennzeichnet,**
**dass** das Zentralrad (2c) des Planetengetriebes (6) mit dem Radring (2a) der Freilaufkupplung (2) verbunden oder einstückig mit diesem ausgebildet ist.

8. Pen-Injektor nach einem der vorherstehenden Ansprüche,
**dadurch gekennzeichnet,**
**dass** Drehbegrenzungsmittel (6c) für das Drehelement (6a) vorgesehen sind, die eine Drehung des Drehelementes (6a) um maximal 360° erlauben.

9. Pen-Injektor nach einem der Ansprüche 4 bis 8,
**dadurch gekennzeichnet,**
**dass** auf dem Radring (2a) des Speicherrades Dosierungszahlen (13) angebracht sind, die durch ein Fenster (12) im Gehäuse (1, 1b) von außen sichtbar sind.

10. Pen-Injektor nach einem der Ansprüche 6 bis 9,
**dadurch gekennzeichnet,**
**dass** auf dem Planetenring (6a) Dosierungszahlen (11) und/oder farbcodierte Sektoren (10) angebracht sind.

11. Pen-Injektor nach Anspruch 9 und 10,
**dadurch gekennzeichnet,**
**dass** die auf dem Radring (2a) angebrachten Dosierungszahlen (13) die Einer-Stellen und die auf dem Planetenring (6a) angebrachten Dosierungszahlen (11) die Zehner-Stellen einer Dosierungsanzeige repräsentieren.

12. Pen-Injektor nach einem der Ansprüche 2-11,
**dadurch gekennzeichnet,**
**dass** das Übersetzungsverhältnis des Übersetzungsgetriebes (6) und die Steigung der Gewindestange (5) so gewählt sind, dass mit einer Umdrehung des Drehelements (6a) der gewünschte Maximalhub erreicht wird.

13. Pen-Injektor nach einem der vorherstehenden Ansprüche,
**dadurch gekennzeichnet,**
**dass** der Kolben (4) aus einem das Gewindeloch (4c) enthaltenden festen Kern (4b) und einer aus nachgiebigem Material bestehenden Umhüllung (4a) besteht.

14. Pen-Injektor nach einem der vorherstehenden Ansprüche,
**dadurch gekennzeichnet**,
das der Behälter (3) für das Injektionsmittel (7) eine Fertigkarpule ist, die die durch den Kolben (4) geführte Gewindestange (5) enthält, und dass der eine Endabschnitt (5b) der Gewindestange (5) über dem Kolben (4) vorsteht.

15. Pen-Injektor nach einem der Ansprüche 2-14,
**dadurch gekennzeichnet,**
**dass** das Gehäuse aus einem becherartigen Unterteil (1a) und einem ebenfalls becherartigen Oberteil (1b) besteht, die mit ihren Öffnungen gegeneinander gerichtet und miteinander verbindbar sind, dass der Unterteil (1a) zur Aufnahme des Behälters für das Injektionsmittel (7) dient, und dass der Oberteil (1b) zur Aufnahme der Freilaufkupplung (2) und des Übersetzungsgetriebes (6) dient.

16. Pen-Injektor nach einem der Ansprüche 2-14,
**dadurch gekennzeichnet,**
**dass** das Gehäuse einen Unterteil (1a) mit mindestens zwei Aufnahmeräumen (1e) für je einen Behälter (3) aufweist, dass für jeden Behälter (3) ein separates Oberteil (1b) vorgesehen ist, welches zur Aufnahme jeweils einer Freilaufkupplung (2) und eines Übersetzungsgetriebes (6) dient, und dass der Unterteil (1a) mit einem Anastomoseteil (15) verbunden ist, welcher die Ausgänge (3a) der Behälter (3) zu einem gemeinsamen Injektionskanal (15f) zusammenführt.

17. Pen-Injektor nach einem der vorherstehenden Ansprüche,
**dadurch gekennzeichnet,**
**dass** er einen Energiespeicher und Antriebsmechanismus (17) aufweist, der mit dem Drehen des Drehelements (6b) in der die Dosierung bewirkenden Drehrichtung geladen wird, und der die Drehung des Drehelementes (6b) zur Ausführung der Injektion in der anderen Drehrichtung bewirkt.

18. Pen-Injektor nach Anspruch 18,
**dadurch gekennzeichnet,**
**dass** der Energiespeicher- und Antriebsmechanismus (17) eine aufgewickelte Wendelfeder (17c) oder dgl. enthält, die bei der die Dosierung bewirkenden Drehung des Drehringes (6b) gestreckt wird, und die sich bei der Ausführung der Injektion auf die ursprüngliche Länge verkürzt.

19. Pen-Injektor nach Anspruch 18,
**dadurch gekennzeichnet,**
**dass** die Wendelfeder (17c) auf einem Wickelkörper (17a) aufgewickelt ist, der am Gehäuse (1) befestigt ist, und dass die Wendelfeder (17c) einerseits am Drehelement (6b) und andererseits am Gehäuse (1c) befestigt ist.

20. Pen-Injektor nach einem der Ansprüche 17 bis 19,
**dadurch gekennzeichnet,**
**dass** der Energiespeicher- und Antriebsmechanismus (17) mit einem von Hand zu betätigenden Auslöseelement (17b) versehen ist.

21. Fertigkarpule für einen Pen-Injektor der gemäß den Ansprüchen 1-20 ausgebildet ist,
**dadurch gekennzeichnet,**
**dass** sie eine durch den Kolben (4) geführte Gewindestange (5) enthält und dass der von der Karpule (3) weggerichtete Endabschnitt (5b) der Gewindestange (5) über dem Kolben (4) vorsteht.

22. Fertigkarpule nach Anspruch 21,
**dadurch gekennzeichnet,**
**dass** der über dem Kolben (4) vorstehende Endabschnitt (5b) der Gewindestange (5) mit sich in axialer Richtung erstreckenden Rastausnehmungen und/ -vorsprüngen (5c) versehen ist.

23. Verfahren zum Betreiben eines Pen-Injektors, der nach einem der Ansprüche 1-20 ausgebildet ist, mit folgenden Schritten:
(aa) das Drehelement (6a) wird - ausgehend vom Drehanschlag (1e) - von Hand zur Dosierung des Hubes für die nächste Injektion in die Richtung gedreht, in der die Freilaufkupplung (2) keine Drehmitnahme bewirkt,
(bb) das Drehelement (6a) wird ebenfalls von Hand oder durch einen Energiespeicher- und Antriebsmechanismus (17) zur Ausführung der Injektion bis zum Anschlag (1e) in die andere Richtung gedreht, in der die Freilaufkupplung (2) eine Drehmitnahme bewirkt.
